# EUROPEAN PATENT APPLICATION

(11) **EP 3 342 338 A1**
(43) Date of publication of application: **04.07.2018**
(21) Application number: 16207321.7
(22) Date of filing: 29.12.2016
(51) Int. Cl.: A61B 5/0408, A61B 5/0428, A61B 5/0478, A61B 5/0492, A61B 5/053, A61N 1/04

(54) **ELECTRODE APPARATUS AND METHOD FOR USING THE SAME**

(71) Applicant: Nokia Technologies Oy, 02610 Espoo (FI)
(72) Inventor: Salo, Antti Oskari, FIN-08500 Lohja as. (FI); Laine, Hannu Ensio, 02750 Espoo (FI); Ikkala, Aleksi, 02610 Espoo (FI)
(74) Representative: Swindell & Pearson Limited

(57) **Abstract**

Certain examples of the present invention relate to a multi-electrode device for use with a user's body part. Certain examples provide an apparatus (100) comprising: a plurality of electrodes (101); a signal line (102) for conveying electrical signals to or from the plurality of electrodes (101); wherein the plurality of electrodes (101) and the signal line (102) are integrally formed in the apparatus (100), and wherein the apparatus (100) is configured such that a user is able to selectively electrically couple one or more of the plurality of electrodes (101) to the signal line (102).

## Description

### TECHNOLOGICAL FIELD

Examples of the present disclosure relate to an electrode apparatus and method for using the same. Some examples, though without prejudice to the foregoing, relate to an apparatus and method for use in biopotential or bioimpedance measurements.

### BACKGROUND

Conventional devices and systems for biopotential or bioimpedance measurements are not always optimal. Their electrodes (which are attached to a user's body part) and connectors for the same may be bulky and may require many free hanging/loose cables which can snag during use such as on a user's clothing. The loose cables can also cause triboelectricity to be generated, which can create unwanted noise in detected signals. Previous devices may not be comfortable during use and may be unsuitable for prolonged use and attachment for an extensive duration. Conventional devices may also be difficult to adapt to differing body part locations and differing sizes of patients.

The listing or discussion of any prior-published document or any background in this specification should not necessarily be taken as an acknowledgement that the document or background is part of the state of the art or is common general knowledge. One or more aspects/examples of the present disclosure may or may not address one or more of the background issues.

### BRIEF SUMMARY

According to at least some examples of the disclosure there is provided an apparatus comprising: a plurality of electrodes; a signal line for conveying electrical signals to or from the plurality of electrodes; wherein the plurality of electrodes and the signal line are integrally formed in the apparatus, and wherein the apparatus is configured such that a user is able to selectively electrically couple one or more of the plurality of electrodes to the signal line.

According to at least some examples of the disclosure there is provided an apparatus comprising: a plurality of electrodes; means for conveying electrical signals to or from the plurality of electrodes;
wherein the plurality of electrodes and said means are integrally formed in the apparatus, and wherein the apparatus is configured such that a user is able to selectively electrically couple one or more of the plurality of electrodes to said means.

The apparatus may be suitable for use in biopotential or bioimpedance measurements.

According to at least some examples of the disclosure there is provided a module or wearable device comprising the above apparatus.

According to at least some examples of the disclosure there is provided a method comprising attaching an apparatus as described above to user's body part; and selectively coupling one or more electrodes to the signal line.

According to at least some examples of the disclosure there are provided embodiments as set out in the appended claims.

### BRIEF DESCRIPTION OF THE DRAWINGS

For a better understanding of various examples of the present disclosure that are useful for understanding the detailed description and certain embodiments of the invention, reference will now be made by way of example only to the accompanying drawings in which:
Figure 1 schematically illustrates an apparatus according to the present disclosure;
Figures 2A, 2B, 2C and 2D schematically illustrate: a bottom view, a plan view, a cross-sectional view, and an in use view of another apparatus according to the present disclosure;
Figures 3A, 3B and 3C schematically illustrate: a plan view, a cross-sectional view, and an in use view of yet another apparatus according to the present disclosure;
Figure 4 schematically illustrates a plan view of yet another apparatus according to the present disclosure;
Figures 5A, 5B, 5C, 5D and 5E schematically illustrate: a bottom layer, a mid layer, a top layer, a cross sectional view and a view of the top layer in use of yet another apparatus according to the present disclosure;
Figures 6A, 6B and 6C schematically illustrate: a bottom view, a top view and a cross sectional view of yet another apparatus according to the present disclosure; and
Figure 7 schematically illustrates a method.

The Figures are not necessarily to scale. Certain features and views of the figures may be shown schematically or exaggerated in scale in the interest of clarity and conciseness. For example, the dimensions of some elements in the figures may be exaggerated relative to other elements to aid explication. Similar reference numerals are used in the Figures to designate similar features. For clarity, all reference numerals are not necessarily displayed in all figures.

### DETAILED DESCRIPTION

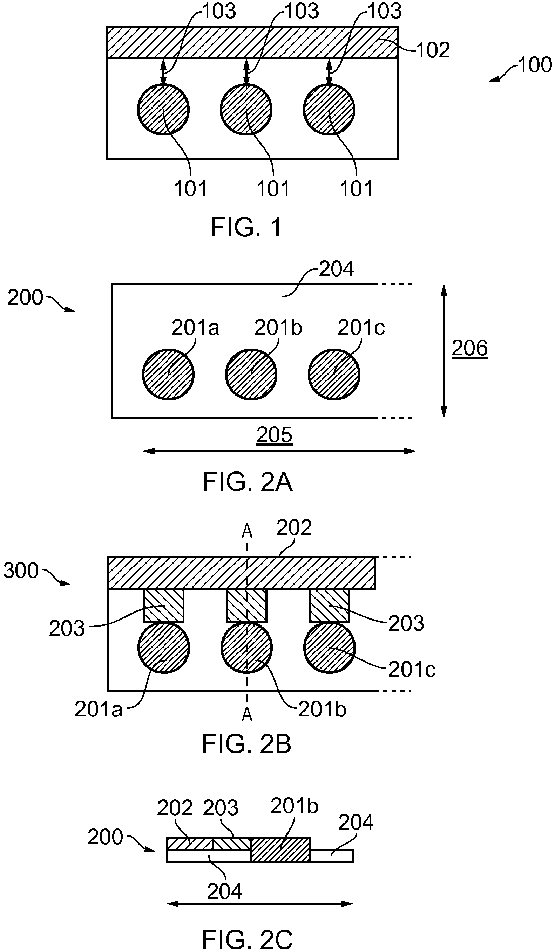

Figure 1 schematically illustrates an apparatus 100 according to the present disclosure and focuses on the functional components necessary for describing the operation of the apparatus.

The apparatus 100 comprises:
a plurality of electrodes 101;
a signal line 102 for conveying electrical signals to or from the plurality of electrodes 101;
wherein the plurality of electrodes 101 and the signal line 102 are integrally formed in the apparatus 100, and wherein the apparatus 100 is configured such that a user is able to selectively electrically couple one or more of the plurality of electrodes 101 to the signal line 102.

As used herein, the term "electrode" may be used to denote an electrical conductor configured for use with a user's body part for conveying electrical signals to or from the user's body part, such as the user's skin/tissue. It may, for example, be a medical electrode, bioelectrode or biological electrode. The electrodes may be for medical purposes, such as for biopotential or bioimpedance measurements. The electrodes may be suitable for use in electrocardiography (ECG), electroencephalography (EEG), electromyography (EMG), impedance cardiography (ICG), bioelectrical impedance analysis (BIA), electrodermal activity (EDA) and galvanic skin response (GSR). In some examples, an electrical signal may be a signal that is detected/picked up by the electrode, e.g. a sensed bioelectrical signal for bio/medical measurements such as those mentioned above. In some examples, an electrical signal may correspond to an electrical impulse/electrical power that is delivered to/applied/injected/imparted to the user's body part, e.g. for therapeutic purposes. The electrode may be integrally formed with the apparatus in that it is structurally integrated into the apparatus so as to be inseparable/non user detachable therefrom.

The signal line 102 may be a conductor, bus or signal wire for conveying electrical signals to or from the plurality of electrodes. The signal line may take the form of a strip-like/band of conducting material or may take the form of a wire. In some examples, in use, the signal line is connected to a signal generator, i.e. to receive electrical signals therefrom for conveying to selected one or more electrodes. In some examples, in use, the signal line is connected to a sensor/measurement device, i.e. for detecting and measuring electrical signals picked up by the selected one or more electrodes. The signal line may be integrally formed with the apparatus in that it is structurally integrated into the apparatus so as to be inseparable/non user detachable therefrom.

The apparatus is configured such that a user is able to selectively electrically couple one or more plurality of electrodes 101 to the signal line 102, as schematically illustrated with reference to arrows 103.

Without limiting the scope of the claims, an advantage/technical effect of some examples of the present disclosure may be to enable the user selection of particular ones of the plurality of electrodes that are to be used, e.g. in biopotential or bioimpedance measurements.

In some examples, such user selection of individual ones of the plurality of electrodes is affected by the removal of one or more conductive elements 203 that, prior to their removal, electrically coupled/connected one or more of the electrodes 101 to the signal line 102. In other examples, the user selection of particular electrodes is achieved by the user application/ user attachment of one or more conductive elements to electrically connect and electrically couple one or more of the electrodes 101 to the signal line 102.

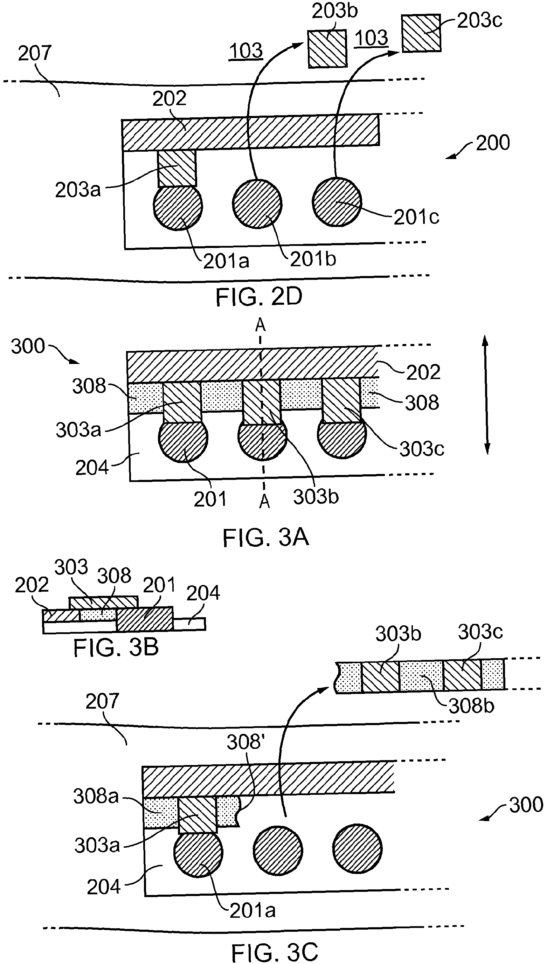

Figures 2A, 2B, 2C and 2D schematically illustrate: a bottom view, a plan view, a cross-sectional view, and an 'in use' view of another apparatus 200 according to the present disclosure.

As shown in the bottom view of figure 2A, the apparatus 200 comprises a substrate/carrier 204 of a non-conductive material. A plurality of electrodes 201a-c are integrated/embedded on a lower surface thereof such that, when in use and the apparatus is attached to a user's body part, the electrodes 201a-c are exposed on a lower exterior surface of the apparatus and are in contact with the user's body part, i.e. in direct physical contact or indirect contact via a conductive medium (such as an electrolyte, conductive colloid, or conductive gel). Alternatively, in some examples, there may be an insulation layer between the electrodes and user's body part, and the electrodes may be capacitively coupled to the user's body part. The insulation layer can be either a part of the apparatus, e.g. integrated with the apparatus, or provided separately of the apparatus, e.g. as a separate external layer such as the user's clothing.

The lower surface of the carrier 204 may be provided with layer of an adhesive medium for affixing the apparatus 200 to a user's body part, such as the user's skin. In some examples, the exposed lower surfaces of the electrodes 201a-c are not provided with an adhesive layer (which could adversely affect the conductivity of the electrode with the user's body part). In some examples, the exposed lower surfaces of the electrodes 201a-c are provided with a conductive adhesive medium (to facilitate both the affixing of the apparatus to the user's body part as well as enhance the electrical coupling between the electrodes and the user's body part).

The apparatus 200 may take the form of an elongate continuous structure (such as a strip, band or a tape) defining a longitudinal lengthwise direction 205 and a lateral widthwise direction 206.

The plan view of figure 2B shows an upper side of the carrier 204 in which the electrodes 201a-c pass through the carrier such that there are exposed upper surfaces thereof for enabling electrical contact with the signal line 202 to be made. The signal line 202 is disposed on the upper surface of the carrier. The electrical contact between the multiple electrodes 201a-c and the signal line 202 is affected by the provision of multiple conductive elements 203. Each conductive element 203 electrically connects an electrode 201a-c to the signal line 202.

The conductive elements 203 are configured to be frangible or removable by a user such that their respective electrode can be user selectively electrically decoupled from the signal line 202, such that the respective electrode is electrically isolated from the signal line and thus unable to convey signals to or receive signals from the signal line 202. Such frangible/removable characteristics of the conductive portion 203 may be provided by any appropriate means, not least such as providing a thin foil-like conductive element which may be readily removed, for example by being scraped, torn or scratched off. Alternatively, the conductive element 203 may comprise one or more weakened/brittle/perforated sections to enable the conductive element 203 to be readily broken and removed.

The conductive elements 203 may be made to be frangible or removable for example via perforation or other lines of weakness, or via the use of a relatively weak adhesive that enables the conductive elements 203 to be peelable/detachable from their underlaying substrate.

Figure 2C shows a cross-sectional view of the apparatus along the line A-A of figure 2B. Figure 2D schematically illustrates the apparatus in use, in particular, schematically showing the removal of various of the conductive elements 203b and 203c (as shown via arrows 103) such that their respective electrodes 201 b and 201 c are electrically decoupled and electrically isolated from the signal line 202 whilst the conductive element 203a remains in position such that its respective electrode 201 a remains electrically connected to the signal line 202.

In the example of Figures 2A-D, the one or more conductive elements 203 are configured to be frangible/removable by a user (e.g. being weakened to enable a user to readily break or remove the conductive element thereby electrically decoupling the one or more electrodes 201 from the signal line 202). However, in certain other examples, conductive elements 203 are not pre-attached to electrically couple the electrodes 201 to the signal line 202. Instead, the one or more of the plurality of electrodes 201 are selectively electrically coupled to the signal line 202 via a user attaching/applying one or more conductive elements to electrically couple the one or more electrodes to the signal line. I.e. the user him or herself adding a conductive element to the apparatus so as to electrically connect an upper exposed portion of the electrode to the signal line 202. For example, in order to select electrode 201 a, instead of removing all of the conductive elements 203b, 203c ... 203n and just leaving conductive element 203a intact, the apparatus may not initially be configured with a plurality of conductive elements electrically connecting their respective electrode to the signal line. The user may his or herself attach conductive element 203a to electrically connect a particular electrode, e.g. 201 a to a signal line 202. Such electrical attachment of a conductive element may be effected by providing a conductive element suitably dimensioned to bridge and electrically connect the electrode to the signal line 202, such a conductive element may further comprise an electrically conductive adhesive to facilitate the affixing of the conductive element and electrical connection between the electrode and signal line whilst maintaining electrical conduction therebetween. Other means for attaching the conductive elements may be provided.

As illustrated by the broken dotted line in figures 2A to 2D, the apparatus 200 may extend in a longitudinal direction 205 and take an elongate form, for example it may take the form of one or more of: a tape, a ribbon, a band, a strip, a strap, a bandage, a multilayered elongate strap, so as to form a continuous tape-like device with multiple selectable electrodes and integrated signal lines/wiring. Such a multi-electrode tape may be simply cut to a desired size. The signal line may form a continuous conductive strip/band extending along the length of the apparatus and the electrodes may form a continuous array along the length of the apparatus. Advantageously, this enables various examples of the apparatus to be readily adaptable to different locations on a user's body and to be attached to differing shapes and contours of the user's body part as well as different sizes and dimensions of users.

The carrier substrate 204 may, in certain examples, comprise an elastic therapeutic tape having elastomeric properties. In such examples, the signal line 202 and/or the electrodes 201 may likewise themselves be configured to have elastomeric properties so as to enable their expansion and contraction along with the expansion and contraction of the elastomeric carrier substrate 204.

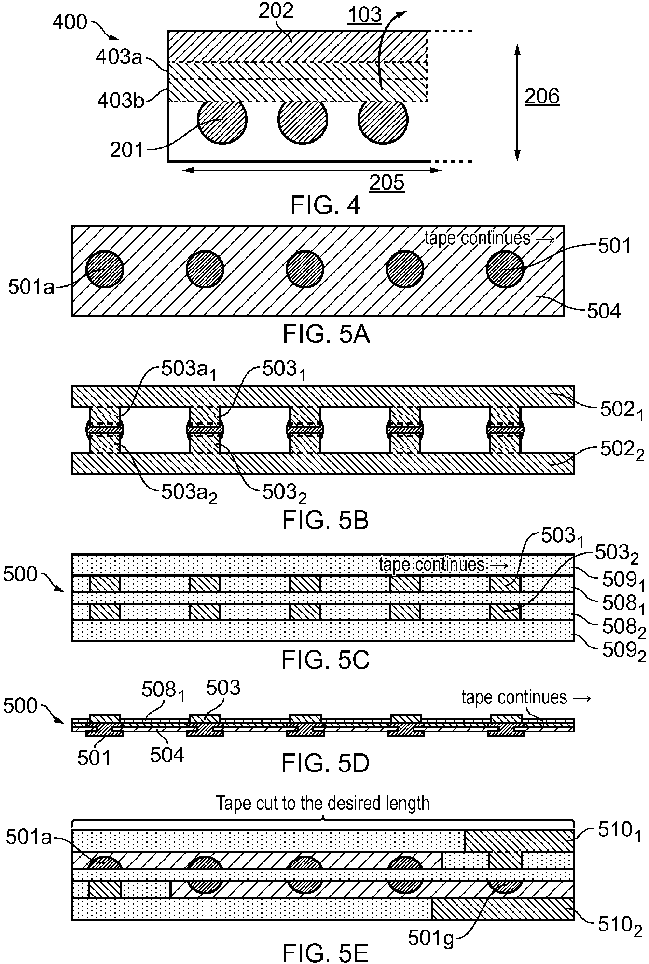

Advantageously, the provision of the apparatus in a tape-like form (e.g. wherein the apparatus is comprised in an elongate flexible structure, carrier or multilayer structure having adhesive on its lower side for attachment to a user's body part) may enable not only adjustable electrode positioning following attachment to a user (e.g. by the subsequent user selection of a particular electrode) but also may enable the user selective adjustment of electrode separation distance (e.g. by selecting a particular further electrode, particularly with regards to examples having two signal lines as per Figures 5A to 6C). Examples of the apparatus may provide a very thin and light weight structure and thus be discrete and unobtrusive. The tape-like structure can be readily cut to a desired length enabling use by persons any size. The integrated signal line may avoid or reduce the need for free-hanging cables and this may avoid/reduce any such free-hanging cables getting snagged/tangled or generating triboelectricity.

The apparatus may be provided in a module. As used here 'module' refers to a unit or apparatus that excludes certain parts/components that would be added by an end manufacturer or a user. For example, the actual measurement device that generates and/or receives signals from the apparatus (e.g. so as to determine biopotential or bioimpedance measurements) may be separately provided and separately attached to the apparatus, for example by an electrical connection/interface with the signal line).

The apparatus may comprise means for affixing the apparatus to a part of the user's body. Such means may comprise use of an adhesive, or by the apparatus being part of a band, strap or bandage that may be wrapped around and secured to a part of a user's body which may involve the use of fasteners such as hook and links or other types of fasteners. This may minimise movement artefacts during signal measurements by preventing relative movement between the electrodes and the user's body part, e.g. tissue.

Figures 3A, 3B and 3C schematically illustrate a plan view, a cross-sectional view and an 'in use' view of an apparatus 300. The apparatus 300 is broadly similar to that of figures 2A to 2D with the exception that the plurality of conductive elements 303a-c are disposed on top of a strip-like elongate member 308, i.e. such that the strip-like elongate member 308 underlies all of the conductive elements 303a-c. With the apparatus 300, instead of a user having to selectively individually remove each of the conductive elements 303 associated with particular electrodes 301 that are not desired to be selected, the apparatus 300 provides an effective way to remove a plurality of the conductive elements 303.

In the apparatus 300, the one or more conductive elements 303a, 303b, 303c are disposed on or affixed to the substrate 308, wherein the substrate itself is configured to be removable or frangible (e.g. via a structural weakness, such as perforations or use of a weak adhesive), such that the removal of the substrate (or one or more parts thereof) removes/breaks the overlaying one or more conductive elements 303, thereby electrically decoupling and deselecting the associated electrodes 301 from the signal line 202. The substrate 308 may be made of a non-conductive material and be configured to be resistant/resilient to tear in a longitudinal direction and may, in some examples, be susceptible to tear in a lateral direction, i.e. so to facilitate its separation into separable parts. For example, with regards to Figure 3C, the substrate 308 may be tearable in a lateral direction, such as is shown with respect to tear line 308' to form two-parts 308a which remains in place and 308b which is removed from the apparatus. Since the conductive elements 303b, 303c ... 303n are disposed on top of the substrate part 308b, the removal of the substrate part likewise removes the conductive elements 303b, 303c ... 303n. Thus, a simplified way of selecting an individual electrode 201 a by leaving conductive element 303a intact and removing the other elements to disconnect their respective electrodes from the signal line is provided in the example of figure 3C.

In the example of figures 3A-C, the frangible/removable conductive elements 303a-c are disposed on top of the substrate 308. In some examples, the conductive elements may be securely affixed (e.g. adhered) to the substrate such that removal of the substrate causes the corresponding removal of the attached conductive elements. In some examples, the substrate may instead be disposed over the conductive portions and the conductive portions may be fixedly attached (e.g. adhered) to a lower surface of the substrate such that removal of the substrate causes a corresponding removal of the attached underlying conductive elements. Such an example may correspond to a strip-like elongate substrate member 308 of figure 3B being disposed on top of and securely attached to the conductive portions 203 of figure 2C.

In the examples described thus far, the conductive elements comprise one or more conductive strips extending in a widthwise direction to electrically connect a single electrode to the signal line. In some examples, a conductive element electrically connects a plurality of electrodes to the signal line. In some examples, such as shown in figure 4, the apparatus 400 may comprise conductive element 403 extending in a longitudinal direction. In the apparatus 400 of figure 4, one or more conductive strips 403a, 403b extend in a longitudinal direction 205. The one or more conductive strips are configured to electrically couple the electrodes 201 to the signal line 202. Each of the one or more conductive elements are frangible or removable by a user such that a user may selectively electrically decouple certain of the electrodes from the signal line, for example by removing/tearing one or more longitudinal/lengthwise sections of the conductive elements, as indicated with reference to arrow 103.

Each longitudinally/lengthwise extending strip 403a, 403b, may electrically couple a plurality (or all) of the electrodes 201 to the signal line 202. Each longitudinally/lengthwise extending strip may extend substantially along the entirety of the length of the apparatus 400.

Figures 5A to 5E schematically illustrate an apparatus 500 comprised of a multilayer structure having an elongate tape-like form.

Figure 5A shows a bottom layer of the apparatus 500 comprising a plurality of electrodes 501 integrated into/embedded within a lower carrier/substrate layer 504. Each electrode may optionally be provided with an electrolyte, in order to facilitate the electrical connection and conduction between the electrodes and a user's skin. The substrate/carrier 504 may comprise an adhesive tape, for example an elastomeric tape or elastic therapeutic tape.

Figure 5B shows an intermediate layer of the apparatus comprising various conductive parts of the apparatus, in particular a first signal line 502₁ and a second signal line 502₂. These signal lines are securely/permanently attached to the apparatus along the entirety of their length, i.e. so as to be integrally formed with the apparatus and inseparable/non user detachable therefrom (as compared to the user detachable conductive elements 503₁ 503₂ that are configured to be selectively detachable (or, in other examples, selectively attachable) to electrically connect an electrode to one of the signal lines. Initially, the apparatus is configured such that each electrode 501 is connected to both of the signal lines 502₁ 502₂. Such electrical connection may be effected by two conductive elements, for example conductive element 503a₁ for electrically connecting electrode 501 a to a first signal line 502₁ and a second conductive element 503a₂ for electrically connecting the electrode 501 a to the second signal line 502₂. Alternatively, a single conductive element may be provided that electrically connects the electrode 501 to each of the signal lines 502₁ and 502₂. Such a single conductive element may be configured to be frangible/removable at least in two sections so as to enable the breaking/removal of two sections of the conductive element to electrically decouple the electrode 501 a from each of the signal lines 502₁ and 502₂.

Figure 5C shows a top view of the apparatus 500 comprising a plurality of removable strips 508₁ and 508₂ of a non-conductive material. Strip 508₁ and 508₂ are similar to the strip 308 of figure 3 in that the conductive portions 503₁ and 503₂ are disposed on top of the underlying strips 508₁ and 508₂ respectively, such that by removing one of the strips (by tearing off a section) any overlaying conductive portions are also removed and their associated electrodes are disconnected from the respective signal line.

In addition to the tear-off strips 508₁ and 508₂ for selecting particular electrodes, further removable strips 509₁ and 509₂ are provided that overlay the signal lines 502₁ and 502₂ respectively. The removable portions 509₁ and 509₂ may be removed so as to reveal and expose an underlying portion of the signal line, for example as shown with respect to figure 5E in which the right hand side sections of the strips 509₁ and 509₂ have been removed so as to reveal and expose the end portions 510₁ and 510₂ of the signal lines 502₁ and 502₂ respectively. Such revealed portions of the signal line may form a terminal of the apparatus for enabling the apparatus to be connected to measurement electronics/circuitry, i.e. for the signal lines to be connected to the measurement electronics/ circuitry.

Figure 5D shows a cross-sectional view of the apparatus 500.

Figure 5E shows the apparatus 500, having been cut to a desired length, in which the electrodes 501 a and 501 g have been selected. In particular, just the electrode 501 a has been selected for connection to the signal line 502₂, by virtue of the removal of the strip 508₂ except for the portion underlying the conductive element 503a₂. Just the electrode 501 g has been selected for electrical connection to the signal line 502₁, by virtue of the removal of the strip 508₁ except for the portion underlying the right-hand side most conductive element.

The apparatus 500 of figures 5A to 5E provides an example of an apparatus comprising two signal lines wherein further one or more conductive elements 503₂ are provided that are configured to electrically couple the one or more electrodes 501 to the second signal line 502₂. Such further conductive elements 503₂ are configured to be frangible or removable so as to selectively electrically decouple their respective electrode from the second signal line.

In certain other examples, the apparatus may comprise yet further additional lines, such as three or more signal lines, and yet further conductive elements for electrically coupling each of the electrodes to the further signal lines. It is to be appreciated that additional non-conductive layers or portions may be provided so as to electrically isolate conductive elements and signal lines from one another, such as is necessary for a particular conductive element to overlay a signal line but without making an electrical connection to the same.

In the multilayer structure shown in figures 5A to 5E, a plurality of electrodes 501 are comprised in a first layer 504 and the signal lines 502₁ and 502₂ are provided in a second layer. In certain examples, the multilayered structure has an elongate form factor with a plurality of electrodes integrated therein forming an array such as row of electrodes extending along the length of the apparatus. Likewise, the signal lines may also be integrally formed in the apparatus and extend along the length of the apparatus.

The plurality of electrodes may be disposed in or on a lower outer surface of the substrate 504, which may be a flexible substrate, forming a first layer. The signal line may be disposed on an upper surface of the first layer so as to form a second layer. The one or more conductive elements may likewise be disposed on the second layer, for example intractably formed with the signal lines, albeit configured so as to be frangible/removable therefrom to electrically decouple the electrodes from the signal lines. Alternatively, the conductive elements may form a third layer disposed over the second layer. One or more strips of non-conductive material may also be provided in the various layers, so as to provide appropriate insulation and electrical isolation, as well as forming tearable strips for facilitating the removal of the conductive elements (discussed with respect to figures 3A to 3C).

The apparatus may be integrated to bandage or therapeutic tape, for example so as to enable monitoring of muscle activity (EMG) in a particular area. The apparatus may be provided with a layer of adhesive on its upper surface so as to enable the attachment of measurement electronics/circuitry to the apparatus. Where a conductive connection is desirable, such as to electrically connect a terminal/connector of the measurement electronics to a signal line, a conductive adhesive material may be used.

Various examples of the apparatus may provide improved user comfort during use and enable prolonged use as the apparatus has a substantially flat form factor and may be adhered to a user's skin (rather than needing to be tightly strapped thereto) via an adhesive that is configured to be skin friendly (i.e. enables easy removal of the apparatus from the user's skin). Yet further, the apparatus may provide a larger surface area in contact with the user's skin than would be provided by separate independent electrodes that are separately attached to a user's skin (and for each of which separate free-hanging/loose cables are required to provide separate individual signal lines for each separate electrode to the remote signal generating/monitoring circuity).

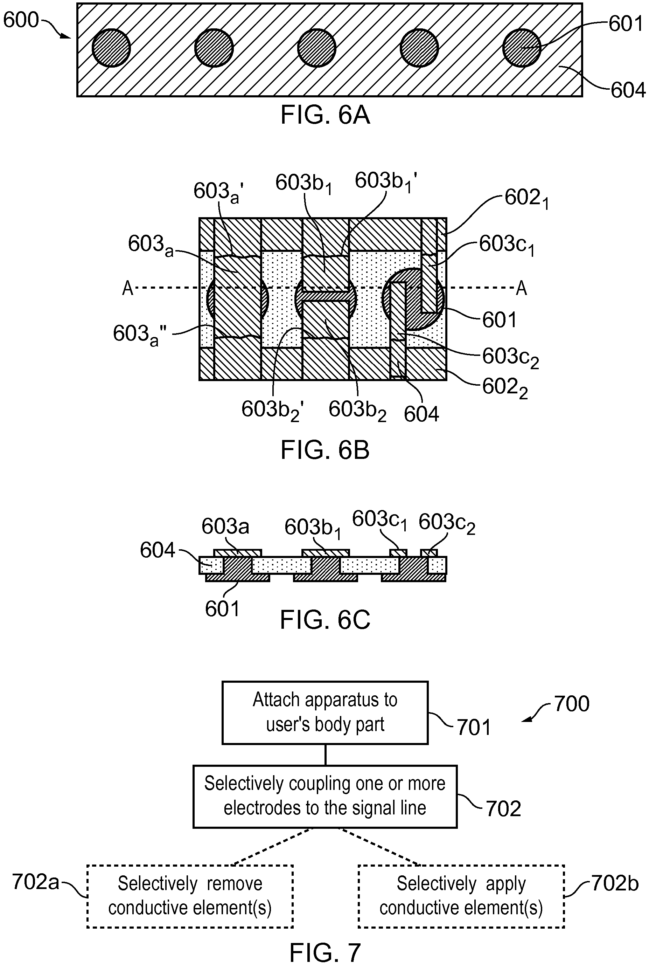

Figures 6A, 6B and 6C schematically illustrate: a bottom view, a top view and a cross-sectional view of a further apparatus 600 that show differing types of configurations and forms of conductive elements that could be implemented in various examples.

The apparatus 600 comprises a multielectrode tape (a section of length thereof which is shown in the figures) wherein a lower surface of a carrier 604 comprises a plurality of electrodes 601. The electrodes have a lower exposed outer surface configured for contacting with a user's skin and an upper surface which passes through the carrier substrate 604 such that the upper surface of the electrode is exposed on an upper surface of the carrier substrate 604. Along a longitudinal length of the apparatus two signal lines are provided 602₁ and 602₂. The two signal lines are configured as two parallel rails between which are the upper surfaces of the electrode. Electrical connection between each electrode and each of the rails may be provided by one or more conductive elements.

Figure 6B shows a selection of possible types of conductive elements that may be used. For example, a single conductive element 603a may be provided that extends from a first signal line 602₁ to a second signal line 602₂ via an intervening electrode so as to electrically connect the electrode to each of the two signal lines. The conductive element 603a may be configured to be frangible/removable at certain sections thereof 603a' and 603a" so as to selectively electrically decouple the electrode from one or other of the signal lines.

Alternatively, an electrode may be coupled to the two signal lines by two conductive elements 603b₁ and 603b₂, each of which electrically connects the electrode to a single signal line, i.e. conductive element 603b₁ electrically connects an electrode to signal line 602₁ and conductive element 603b₂ electrically connects the electrode to signal line 602₂. Again, frangible/removable sections may be provided to each of the conductive elements, namely sections 603b₁' and 603b₂' respectively, so as to enable the selective electrically decoupling of the electrode from each of the signal lines 601₁ and 602₂.

Alternative types of conductive elements 603c₁ and 603c₂ may be provided which are narrower than conductive elements 603b₁ and 603b₂. Such narrow conductive elements may enable the end portions of the respective conductive elements to extend past one another on the upper surface of the electrode (as compared to conductive elements 603b₁ and 603b₂ whose end portions can merely proximately abut one another).

Figure 7 schematically illustrates a flow chart of a method 700 according to an example of the present disclosure.

In block 701, an apparatus as described above (e.g. with respect to any of apparatuses 100, 200, 3000, 400, 500 and 600) is attached to a user's body part. In block 702 one or more of the electrodes are selectively coupled to the signal line.

Such selective coupling of the one or more electrodes to the signal line may comprise, as shown in block 702a, selective user removal of one or more conductive elements, or, as shown in block 702b, the selective user application of one or more conductive elements.

The flowchart of Figure 7 represents one possible scenario among others. The order of the blocks shown is not absolutely required, so in principle, the various blocks can be performed out of order. For example, the user selective coupling of one or more of the electrodes to the signal line may be carried out prior to the apparatus being attached to a user's body part.

In certain examples one or more blocks may be performed in a different order or overlapping in time, in series or in parallel. One or more blocks may be omitted or added or changed in some combination of ways. For example, the selective coupling of one or more electrodes to the signal line may involve both the selective removal of certain conductive elements as well as the selective application of other conductive elements.

Features described in the preceding description may be used in combinations other than the combinations explicitly described. Although functions have been described with reference to certain features, those functions may be performable by other features whether described or not. Although features have been described with reference to certain examples, those features may also be present in other examples whether described or not.

Although various examples of the present disclosure have been described in the preceding paragraphs, it should be appreciated that modifications to the examples given can be made without departing from the scope of the invention as set out in the claims. For example it will be readily apparent that different shapes and dimensions of the various components may be provided. For example, whilst generally circular electrodes have been shown, electrodes of other shapes may be provided. Likewise, whilst generally elongate rectilinear strip/band-like sections of signal lines have been shown, alternative shape and dimensions of signal lines may be provided. Whilst the electrodes have been shown passing through a lower substrate/carrier of the apparatus, the electrode may be provided on a lower surface of the lower substrate/carrier and an electrical via may be used that passes through the lower substrate/carrier to the upper surface thereof to enable electrical connection to a conductive element for electrically connecting the electrode to or from a signal line. Whilst apparatuses for a one lead measurement (i.e. with two selected electrodes, one selectively coupled to a first signal line and another electrode selectively coupled to a second different signal line) have been described, it is to be appreciated that the apparatus could be implemented in multi-lead configurations, for example a grounded three electrode and multi-lead configurations may be provided by the addition of further signal lines. Insulating material can be provided when conductive elements cross a signal line.

In certain examples, three electrode/three lead functionality for a measuring device may be provided by using a two signal line apparatus by: attaching a first terminal/connector of the measuring device to the first signal line (the first signal line being electrically coupled to at least a first electrode), attaching a second terminal/connector of the measuring device to the second signal line (the second signal line being electrically coupled to at least a second electrode), and attaching a third terminal/connector of the measuring device to another electrode.

Various of the examples discussed above show only a single electrode being selected for connection to a signal line. It is to be appreciated that the apparatus may enable two or more of the electrodes to be connected to the same signal in use, thus providing, in effect, a larger electrode area for the measurement. The two or more selected electrodes could be any individual electrodes of the array (i.e. they need not necessarily be adjacent/neighbouring electrodes in the array).

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one ..." or by using "consisting".

In this description, the wording 'connect', 'couple' and 'communication' and their derivatives mean operationally connected/coupled/in communication. It should be appreciated that any number or combination of intervening components can exist, e.g. electrolytes and/or adhesive materials where appropriate, (including no intervening components).

In this description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some or all other examples. Thus 'example', 'for example' or 'may' refers to a particular instance in a class of examples. A property of the instance can be a property of only that instance or a property of the class or a property of a sub-class of the class that includes some but not all of the instances in the class.

In this description, references to "a/an/the" [feature, element, component, means ...] are to be interpreted as "at least one" [feature, element, component, means ...] unless explicitly stated otherwise.

The above description describes some examples of the present disclosure however those of ordinary skill in the art will be aware of possible alternative structures and method features which offer equivalent functionality to the specific examples of such structures and features described herein above and which for the sake of brevity and clarity have been omitted from the above description. Nonetheless, the above description should be read as implicitly including reference to such alternative structures and method features which provide equivalent functionality unless such alternative structures or method features are explicitly excluded in the above description of the examples of the present disclosure.

Whilst endeavouring in the foregoing specification to draw attention to those features of examples of the present disclosure believed to be of particular importance it should be understood that the applicant claims protection in respect of any patentable feature or combination of features hereinbefore referred to and/or shown in the drawings whether or not particular emphasis has been placed thereon.

The examples of the present disclosure and the accompanying claims may be suitably combined in any manner apparent to one of ordinary skill in the art.

## Claims

1. An apparatus comprising:
a plurality of electrodes;
a signal line for conveying electrical signals to or from the plurality of electrodes;
wherein the plurality of electrodes and the signal line are integrally formed in the apparatus, and wherein the apparatus is configured such that a user is able to selectively electrically couple one or more of the plurality of electrodes to the signal line.

2. The apparatus of claim 1, further comprising one or more conductive elements configured to selectively electrically couple the one or more electrodes to the signal line.

3. The apparatus of claim 2, wherein the one or more conductive elements are configured to electrically couple the one or more electrodes to the signal line, and wherein the one or more conductive elements are configured to be frangible or removable by a user such that the one or more electrodes are user selectively electrically decouplable from the signal line.

4. The apparatus of any one or more of previous claims 2 to 3, wherein the one or more conductive elements are disposed on or affixed to one or more parts of a substrate, wherein the one or more parts of the substrate are configured to be frangible or removable, such that removal of the one or more parts of the substrate removes the one or more conductive elements.

5. The apparatus of any one or more of the previous claims, wherein the apparatus is comprised in an elongate tape-like flexible structure.

6. The apparatus of claim 5, wherein the signal line extends substantially entirely along the length of the apparatus.

7. The apparatus of claim 5 or 6, wherein the one or more conductive elements comprises:
one or more conductive strips extending in a widthwise direction, or
one or more conductive strips extending in a longitudinal direction.

8. The apparatus of any one or more of the previous claims, wherein the apparatus comprises at least a second signal line integrated with the apparatus for conveying electrical signals to or from the plurality of electrodes, and wherein the apparatus is configured such that one or more of the plurality of electrodes is user selectively electrically couplable to the at least second signal line.

9. The apparatus of any one or more of the previous claims, wherein the apparatus comprises a multilayered structure, wherein the plurality of electrodes are comprised in a first layer and wherein the signal line is comprised in a second layer.

10. The apparatus of any one or more of the previous claims, further comprising one or more portions of a non conductive material, wherein the non conductive material is configured to be selectively removable from the apparatus.

11. The apparatus of any one or more of the previous claims, wherein the apparatus comprises means for affixing the apparatus to a part of a user's body.

12. The apparatus of any one or more of the previous claims, wherein the apparatus is configured for use in biopotential or bioimpedance measurements.

13. A module or device comprising the apparatus of any one or more of previous claims 1 to 12.

14. A method comprising:
attaching an apparatus according to any of claims 1 to 12 to user's body part; and
selectively coupling one or more electrodes to the signal line.

15. The method of claim 15, wherein:
the apparatus comprises one or more conductive elements configured to electrically couple the one or more electrodes to the signal line, and wherein the selective coupling comprises selectively removing at least one of the one or more conductive elements; or
the selective coupling comprises selectively applying one or more conductive elements to electrically couple the one or more electrodes to the signal line.
